(19) **Europäisches Patentamt European Patent Office Office européen des brevets**

(11) **EP 3 226 773 B1**

(12) **EUROPEAN PATENT SPECIFICATION**

(45) Date of publication and mention
of the grant of the patent:
**25.10.2023 Bulletin 2023/43**

(21) Application number: **16700777.2**

(22) Date of filing: **04.01.2016**

(51) International Patent Classification (IPC):
***A61B 8/08*** *(2006.01)* ***A61B 8/12*** *(2006.01)*

(52) Cooperative Patent Classification (CPC):
**A61B 8/085; A61B 8/0883; A61B 8/12;**
**A61B 8/445; A61B 8/485; A61B 8/5223;**
**A61B 8/543; G16H 50/30**

(86) International application number:
**PCT/US2016/012031**

(87) International publication number:
**WO 2016/130234 (18.08.2016 Gazette 2016/33)**

(54) **SYSTEMS FOR LESION FORMATION FEEDBACK**

SYSTEME FÜR VERLETZUNGSBILDUNGSFEEDBACK

SYSTÈMES DE RÉTROACTION SUR LA FORMATION DE LÉSIONS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **09.02.2015 US 201562113833 P**

(43) Date of publication of application:
**11.10.2017 Bulletin 2017/41**

(73) Proprietor: **St. Jude Medical, Cardiology Division,
Inc.**
**St. Paul, MN 55117 (US)**

(72) Inventors:
• **SLIWA, John W.**
**San Jose, California 95110 (US)**

• **MA, Zhenyi**
**Santa Clara, California 95050 (US)**

(74) Representative: **Alpspitz IP**
**Longinusstraße 1**
**81247 München (DE)**

(56) References cited:
**WO-A1-03/075771       JP-A- 2012 170 777**
**US-A1- 2010 113 985   US-A1- 2010 198 065**
**US-A1- 2011 257 523   US-A1- 2012 265 069**
**US-A1- 2012 265 069   US-A1- 2014 081 262**

**Description**

BACKGROUND

The instant disclosure relates generally to tissue ablation. In particular, the instant disclosure relates to systems, apparatuses, and methods for monitoring the formation of a lesion, for example in cardiac tissue, using echography.

Catheters are used in a variety of diagnostic and therapeutic procedures, for example to diagnose and/or treat conditions such as atrial arrhythmias. For example, a catheter carrying one or more electrodes can be deployed and manipulated through a patient's vasculature and, once located at the intended site, radiofrequency ("RF") energy can be delivered through the electrodes to ablate tissue.

In some catheters, an additional sensor, such as an ultrasound sensor, is provided in the catheter tip to provide additional information during the primary diagnosis or therapy. For example, practitioners often desire information about lesion formation, such as lesion depth, lesion pop-potential, and lesion transmurality. Thus, RF ablation catheters can include one or more ultrasound sensors, located within the hollow tip of the catheter, that can be used to monitor the progress of a lesion forming in the tissue being treated and/or to confirm one or more characteristics of the lesion once created.

US 2010/0113985 A1 relates to systems and methods for ablating tissue, in particular to the treatment of fibrillation or other arrhythmias of the heart by using ultrasound energy.

US 2010/0198065 A1 relates to systems and methods for creating ablation zones in human tissue.

US 2014/0081262 A1 relates to analysing anatomical structures within a body by characterizing the condition of tissue as part of a cardiac map.

US 2012/0265069 A1 relates to acoustic transducers for lesion feedback, catheter tip contact-monitoring, tissue thickness measurement, and pre-pop warning.

The invention is defined by the appended claims, all other embodiments are merely exemplary.

In an embodiment, a system for measuring lesion formation in a tissue includes a lesion analysis processor programmed to receive as input at least two A-line scan echograms of the tissue, to determine progress of a lesion forming in the tissue from the at least two A-line scan echograms, and to output feedback about the lesion. The lesion analysis processor can determine the progress of the lesion forming in the tissue using one or more approaches described herein (*e.g.*, changes in brightness from echogram to echogram; changes in tissue elasticity as evidenced by changes in acoustic reflector movement between echograms; tissue shrinkage as evidenced by acoustic reflector movement between echograms; changes in resonant microbubble distribution from echogram to echogram).

The foregoing and other aspects, features, details, utilities, and advantages of the present invention will be apparent from reading the following description and claims, and from reviewing the accompanying drawings.

BRIEF DESCRIPTION OF THE DRAWINGS

Figure 1 depicts an ablation and lesion feedback system including an exemplary catheter including a sensor-bearing tip.

Figure 2 is a transverse cross-section of a sensor-bearing catheter tip according to a first aspect disclosed herein. Portions of the tip are rendered transparent in order to illustrate details of the interior thereof.

Figure 3 is a perspective view of a sensor-bearing catheter tip according to a second aspect disclosed herein. Portions of the tip are rendered transparent in order to illustrate details of the interior thereof.

Figure 4 is a perspective view of a sensor-bearing catheter tip according to a third aspect disclosed herein. Portions of the tip are rendered transparent in order to illustrate details of the interior thereof.

Figure 5 is a perspective view of a sensor-bearing catheter tip according to a fourth aspect disclosed herein. Portions of the tip are rendered transparent in order to illustrate details of the interior thereof.

Figure 6 is a perspective view of a sensor-bearing catheter tip according to a fifth aspect disclosed herein. Portions of the tip are rendered transparent in order to illustrate details of the interior thereof.

Figure 7 schematically illustrates the formation of a lesion.

Figure 8 is a flowchart of representative steps that can be carried out to monitor the formation of a lesion according to a first method disclosed herein.

Figures 9a and 9b illustrate the application of the lesion formation monitoring method represented by Figure 8.

Figure 10 is a flowchart of representative steps that can be carried out to monitor the formation of a lesion according to a second method disclosed herein.

Figure 11 is a flowchart of representative steps that can be carried out to monitor the formation of a lesion according to a third method disclosed herein.

Figure 12 is another schematic illustration of the formation of a lesion.

Figure 13a illustrates a narrowband acoustic pulse that can be utilized to monitor the formation of a lesion according to a fourth method disclosed herein.

Figure 13b illustrates the acoustic echoes resulting from the narrowband acoustic pulse shown in Figure 13a when applied in the arrangement depicted in Figure 12.

DETAILED DESCRIPTION

The present disclosure provides methods, apparatus, and systems for monitoring the formation of a lesion in tissue. For purposes of illustration, several exemplary embodiments will be described herein in detail in the context of a radiofrequency ("RF") ablation catheter including an acoustic sensor (e.g., a pulse-echo transducer; a photoacoustic transducer) that can be used to monitor the progress of the lesion being formed in an adjacent tissue. It should be understood, however, that the methods, apparatuses, and systems described herein can be utilized in other contexts.

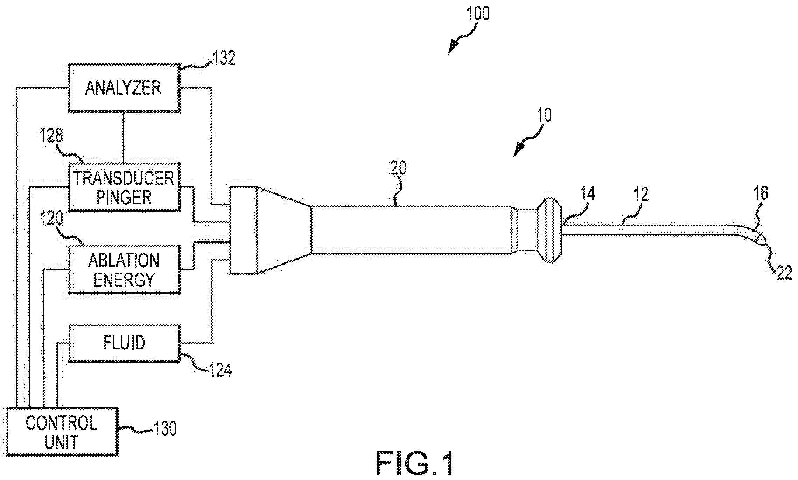

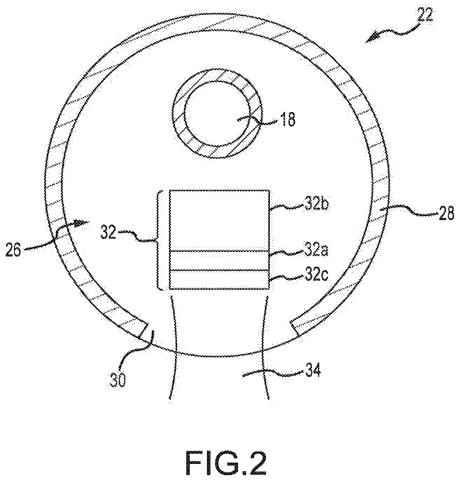

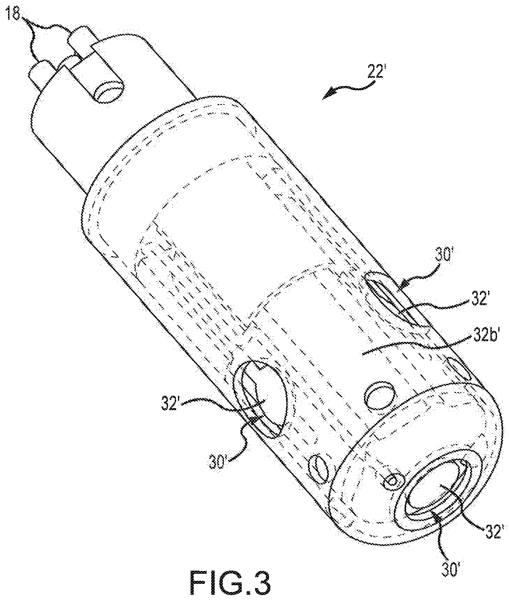

Figure 1 is a schematic diagram of an ablation and lesion feedback system 100 including an exemplary catheter 10. As shown in Figure 1, catheter 10 generally includes an elongate, hollow, and flexible tubular body 12 having a proximal end 14 and a distal end 16. Tubular body 12 defines a lumen 18 (not visible in Figure 1, but visible, *inter alia,* in Figure 2). Although only a single lumen 18 is depicted in certain figures for clarity of illustration, it should be understood that any number of lumens 18 can be used without departing from the scope of the instant teachings (*see, e.g.,* multiple lumens as depicted in Figure 3). As the person of ordinary skill in the art will appreciate, tubular body 12 can also contain electrical interconnect wires, pull-wires, thermocouple leads, and the like.

Proximal end 14 of tubular body 12 is attached to a catheter control handle 20. Catheter control handle 20 can include, for example, an actuator (not shown) coupled to suitable structure (*e.g.*, pull wires and/or pull rings) within tubular body 12 in order to effect the deflection of distal end 16 in one or more bending planes. It can also include electrical power and/or signal connections to additional components of ablation system 100 as discussed in further detail below.

A hollow tip 22 is attached to distal end 16 of tubular body 12. As used herein, the term "hollow" means that tip 22 includes at least one cavity, for example to contain ultrasound transducer 32 as described below. Various suitable embodiments of tip 22 will be described in further detail below with reference to Figures 2-6. In general, however, for purposes of the instant disclosure, tip 22 can include an RF ablation element, such as a tip electrode, and can, as such, be connected with an ablation energy source 120, such as an RF generator.

Figure 2 is a cross-sectional view of hollow tip 22 according to certain aspects of the instant disclosure. As shown in Figure 2, an irrigant lumen 18 of tubular body 12 is in fluid communication with the interior 26 of hollow tip 22, which is defined by a wall 28 of hollow tip 22. An irrigant (*e.g.*, saline) or other fluid can be delivered from fluid source 124 (shown in Figure 1), through lumen 18, and into hollow tip 22, for example for cooling purposes, for energy transmission purposes, and/or for acoustic matching purposes.

Wall 28 of hollow tip 22 further includes a window (or "beam hole") 30 (*e.g.,* a break in wall 28). Window 30 allows for the passage of acoustic energy to and/or from a sensor, such as pulse/echo ultrasound transducer 32 disposed within interior 26 of hollow tip 22, along an acoustic beam path 34. As seen in Figure 2, and as will be familiar to the person of ordinary skill in the acoustic art, transducer 32 can include a piezomaterial layer 32a, an attenuative backer material 32b, and one or more acoustic matching layers 32c. It should also be understood that, as used herein, the term "transducer" encompasses all manner of ultrasound transducers, including, without limitation, single element transducers, multielement transducers, focused transducers, unfocused transducers, transducers with acoustic matching layers 32c, transducers without acoustic matching layers 32c, piezomaterial-based (*e.g.,* PZT, PVDF) transducers, photoacoustic-based transducers, thermoacoustic based transducers, and capacitive micromechanical ultrasound transducers ("CMUT").

Window 30 also allows for irrigant to pass out of interior 26 of hollow tip 22, for example for tissue and transducer cooling purposes, acoustic energy transmission purposes, and/or for ablation energy coupling to adjacent tissue. The irrigant, such as saline, can benefit the coupling and transmission of both RF ablation energy and pulse-echo acoustic energy to (and, in the case of pulse-echo energy, from) the adjacent tissue.

A transducer pinger 128 (*see* Figure 1), which might have more than one transducer channel, supplies pinging energy, such as electrical energy pulses, to transducer 32, resulting in the generation of an acoustic wave along beam path 34 as shown in Figure 2. A control unit 130 (also shown in Figure 1) is provided for controlling the ablation and the acoustic pinging during ablation. For instance, control unit 130 can be configured to carry out duty cycling or synchronization for both ablation and pinging. (The term "pinging" is used herein to mean transmitting acoustic energy and then receiving the reflected or echoed acoustic energy.)

An acoustic pinger echo analyzer or acoustic receiver 132 is provided to condition and analyze the data collected by transducer 32 to provide lesion feedback. The information can be presented to a practitioner (*e.g.,* using a graphical user interface) to provide real-time assessment of the ablation target, the ablation process, and/or the ablation result. The information may additionally or alternatively be used by the system itself without operator intervention, for example as input to a feedback control loop controlling ablation power and/or irrigant cooling to avoid steam pops and/or to achieve a desired lesion depth.

Thus, one aspect disclosed herein is directed to an RF ablation catheter with one or more ultrasound transducers therein or thereon, wherein the transducer is capable of, *inter alia,* acoustic lesion feedback. The catheter is capable of delivering an RF ablating tip to a patient's tissue to be ablated. These aspects and others are described in United States patent application publication no. 2012/0265069.

Figure 3 illustrates another embodiment of a tip, designated 22', suitable for use in connection with the teachings herein. Tip 22' includes four transducers 32' arranged in what can be referred to as a "3+1" arrangement. Three such transducers 32' are oriented with their beam paths (and corresponding windows 30') at about 90 degrees to the longitudinal axis of tip 22' (*e.g.*, side-looking) and at about 120 degrees from each other about the circumference of tip 22' (only two of the side-looking transducers 32' are visible in Figure 3, with the third side-looking transducer 32' on the underside of hollow tip 22'). The fourth transducer 32' is oriented with a forward axially-directed beam path (and corresponding window 30') substantially parallel to the longitudinal axis of tip 22' (*e.g.,* forward-looking).

Transducers 32' in tip 22' can be attached to a common attenuative backer 32b'. Backer 32b' can be made of an injection-molded polymer containing tungsten particles; the polymer can be, for example, an amorphous thermoplastic polyetherimide (*e.g.*, Ultem™), poly ether sulfone ("PES"), or another strong, creep-resistant, high-temperature polymer material such as poly ether ether ketone ("PEEK"). Injection molding at high pressure and temperature can advantageously utilize molding pellets that already contain the desired concentration of tungsten particles to achieve the desired acoustic attenuation and acoustic impedance of backer 32b'. Moreover, the high-temperature, high-strength nature of the polymer allows for transducer lamination and for some deposited or laminated electrodes to be deposited on backer 32b' at modest processing temperatures.

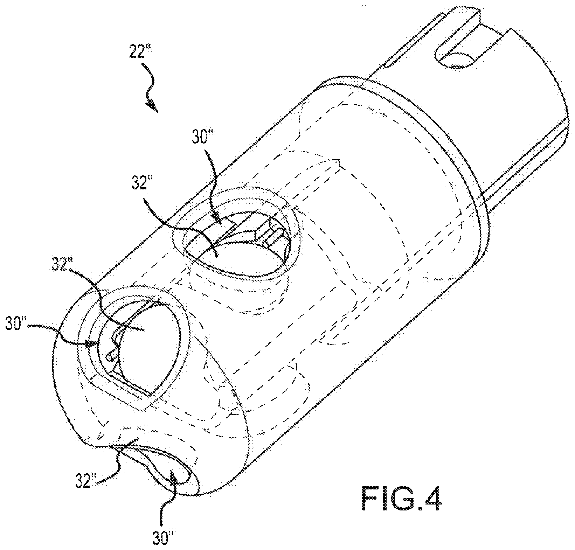

Figure 4 is yet another embodiment of a tip 22" suitable for use in connection with the teachings herein. Tip 22" likewise includes four transducers 32" arranged in what can be referred to as a "2+2" arrangement. Two transducers 32" are oriented with their beam paths (and corresponding windows 30") at about 45 degrees to the longitudinal axis of tip 22"; they are therefore partially forward-looking and partially side-looking (referred to herein as "forward/side-looking"). The other two transducers 32", which can be oriented proximally of the forward/side-looking transducers 32", are oriented to be primarily side-looking, and can be spaced about 180 degrees from each other about the circumference of hollow tip 22".

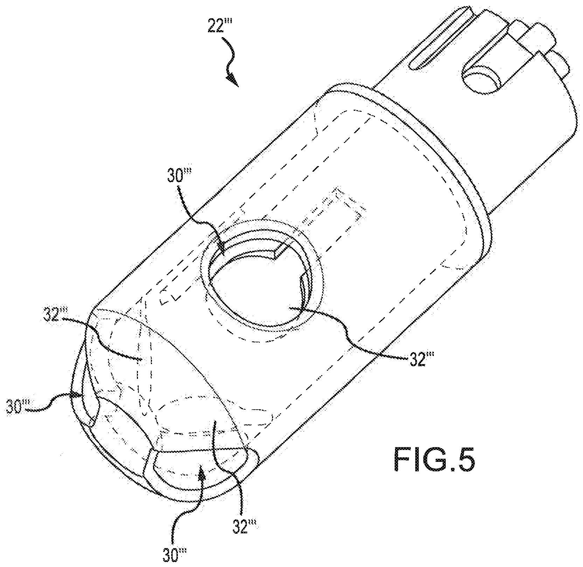

A variation on the 2+2 arrangement is shown as tip 22‴ in Figure 5. In Figure 5, the forward/side-looking transducers 32‴ are rotated about 90 degrees about the longitudinal axis of hollow tip 22‴ relative to the orientation shown in Figure 4. Thus, in tip 22‴, each of the forward/side-looking transducers 32‴ has a beam path in a unique plane, whereas, in the embodiment of Figure 4, all four transducers 32" have their beam paths in a common plane.

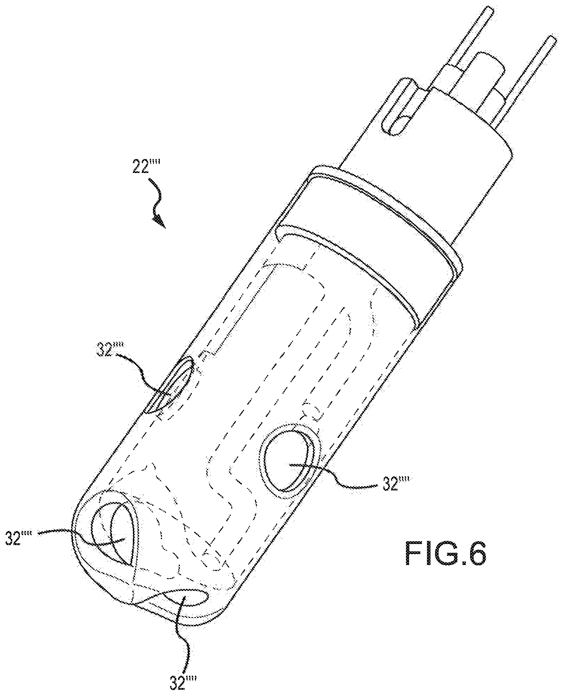

A further embodiment of a tip, designated 22"", is shown in Figure 6. In tip 22"", there are five transducers 32‴′ oriented in what can be referred to as a "3+2" arrangement. In particular, two transducers 32"" are oriented to be forward/side-looking, while three transducers 32‴′ are oriented to be side-looking.

It should be understood that the embodiments depicted in Figures 4-6 can likewise utilize a common attenuative backer analogous to backer 32b' of Figure 3.

Other arrangements are also contemplated. For example, the side-looking transducers can be removed from the configurations shown in Figures 4-6, leaving only the forward/side-looking transducers.

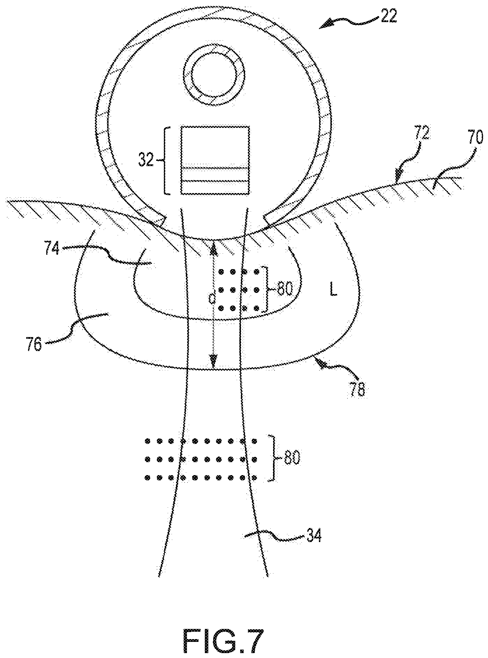

Figure 7 depicts a lesion (denoted "L") forming in a tissue 70, such as a cardiac tissue, for example as a result of RF energy delivered by catheter 12. As those of ordinary skill in the art will appreciate, lesion L will typically include a core region 74, which is slightly browned, and a surrounding region 76, which is white upon sectioning. Lesion L is defined by a lesion front 78, which extends a depth "d" below tissue surface 72. Although lesion front 78 will advance through tissue 70 as additional RF energy is applied, at any given time, tissue beyond lesion front 78 is substantially unaffected (*e.g.,* it has its inherent naturally high elasticity and low ultrasonic reflectivity).

Transducer 32 can be used to monitor the formation of lesion L according to various methods and aspects disclosed herein. In general, these aspects compare echograms acquired at different times (*e.g.,* before any ablation, after some incremental ablation, after ablation is fully complete) to measure the formation of lesion L. It is also desirable for the echograms to extend depthwise from the tissue surface 72 to a depth beyond the desired final depth d of lesion L, such that an unablated region remains "underneath" (that is, below or behind) the desired lesioned region L in the echogram. Although several embodiments are disclosed herein in connection with A-line scan echograms, other ultrasound imaging modes may be employed in other embodiments to analyze tissue 70.

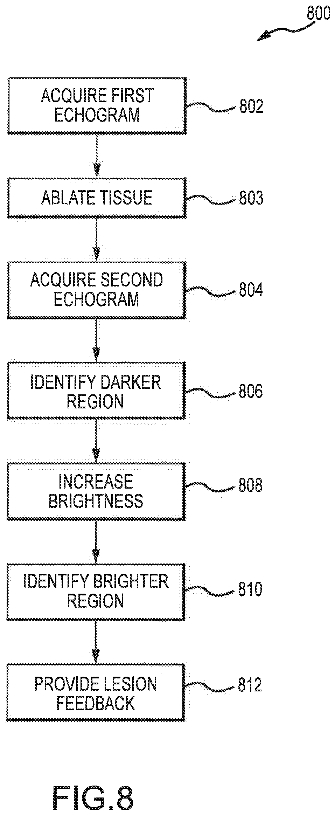

Figure 8 is a flowchart 800 of representative steps that can be carried out to measure the formation of lesion L according to a first aspect of the disclosure. In block 802, a first echogram (*e.g.,* a first A-line scan) of tissue 70 is acquired, and optionally saved, for example using transducer 32 operating at a first transmit power and a first gain.

In block 804, a second echogram (*e.g.*, a second A-line scan) of tissue 70 is acquired at a later time, for example following at least some incremental ablation of tissue 70 in block 803. As used herein, the term "incremental ablation" means that the final overall depth and/or volume of lesion L is formed in discrete depth and/or volume increments, allowing for the teachings herein to be applied between increments, for example to monitor lesion progress and/or adjust power, time, and/or other operating parameters for future ablation increments. An incremental ablation as disclosed herein can be very fine (*e.g.,* 100 or more increments to create the full depth and/or volume) or very coarse; indeed, it can even be completed in a single increment.

Like the first echogram, the second echogram can be acquired using transducer 32 and optionally saved. It is also desirable for the second echogram to be acquired with tip 22 in substantially the same position and rotational orientation with respect to the beating heart as it was in when acquiring the first echogram, as can be done by synchronizing the pinging of transducer 32 with the heartbeat, so that both echograms capture substantially the same scan line through tissue 70.

As those of ordinary skill in the art will appreciate, as lesion L is formed, an echogram of tissue 70 can exhibit brighter or higher-amplitude contrast due, *inter alia,* to micro bubbling and tissue protein crosslinking in region L, particularly at near-field depths less than d. The growing microbubble cloud at more superficial depths, however, can prevent ultrasound from penetrating tissue 70 more deeply, which leads to the deeper tissue appearing darker in echograms taken after lesioning (*e.g.,* the second echogram) than in echograms taken before lesioning (*e.g.,* the first echogram). In other words, the formation of microbubbles due to heated tissue at shallower depths masks the cooler tissue's own inherent brightness or contrast at deeper depths. This darkened, unablated and unbubbled region is identified in block 806. The ablation practitioner will also appreciate that, although much of the darkening of deeper tissue is due to nearer-surface microbubbles, some of it is attributable to protein crosslinking in the hot tissue.

In block 808, the brightness (*e.g.,* the amplitude gain) of the second echogram is increased to restore the original brightness of the darkened unablated deeper region. As used herein, the term "brightness" refers to the amplitude of the received echo from a given point in tissue 70, such as at a particular depth along a particular scan line at such an unablated depth greater than d. For example, the brightness or echo amplitude can be increased at all depths by increasing the logarithmic signal gain amplitude of the second echogram or by operating transducer 32 at a higher transmit power and/or with an increased gain relative to the first echogram until the deeper, darkened, unlesioned tissue is restored to its original brightness or reflective amplitude. Increasing the transmit power of transducer 32 may be somewhat more desirable than increasing gain, as increasing gain can introduce noise into the acquired echogram. In the interest of time, coarse adjustments to transmit power and/or gain can be made prior to acquiring the second echogram based on experience.

When the deeper, darkened unablated region of the second echogram is restored to its original preablation brightness (*i.e.,* the brightness or echo-amplitude versus depth of the first echogram), the brightness of the more superficial region of the second echogram will also increase, ultimately appearing brighter in the second echogram than the first echogram. This brighter region is identified in block 810.

In block 812, the brighter-depth region identified in block 810 is used to provide feedback about lesion L forming in tissue 70. The brighter region will typically start at or near tissue surface 72 and extend to tissue that has no microbubbling and no crosslinking (*i.e.,* tissue that is unburned and not very hot). For example, in some embodiments, the depth of the brighter region can be provided to a user directly as the depth d of lesion L. In other embodiments, the depth d of lesion L can be extrapolated from the depth of the brighter region, for example by multiplying the depth of the brighter region by a correction factor that can be experimentally determined for a given transducer and/or operating frequency.

The foregoing steps can also be repeated, either at the same location as lesion L continues to form incrementally, or at a different location. In the case where the foregoing steps are repeated, the later-acquired echogram can be compared to the originally-acquired echogram, the immediate prior echogram, or to a composite or average thereof.

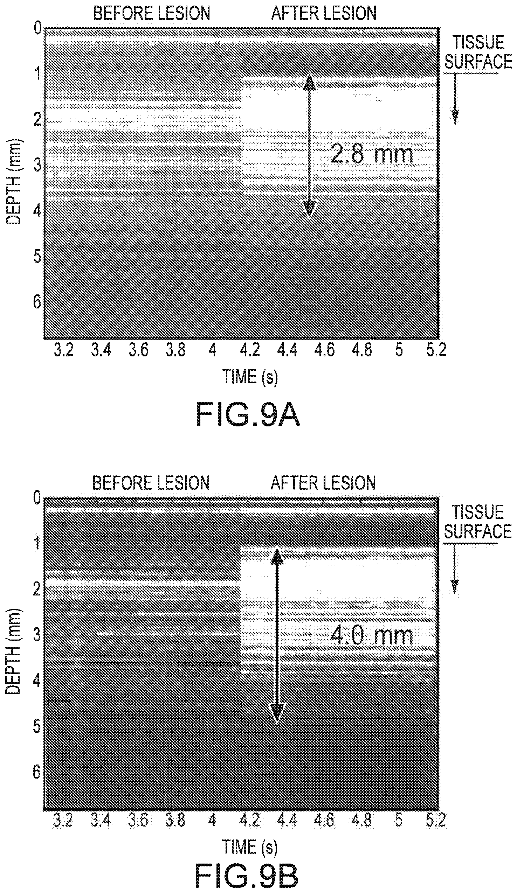

Figures 9a and 9b are A-line scan echograms that illustrate the use of the method described above to identify the depth of a lesion. Starting from the left-hand side of each figure (around 3 seconds) are quick echograms taken prior to any ablation. The right-hand side of each figure (starting around 4.2 seconds) is a post-ablation quick echogram in the same tissue location. In Figure 9a, one can see the darkened region described above prior to any brightness adjustment. In Figure 9b, the darkened region has been restored to its previous (*i.e.,* pre-ablation) brightness, which has also increased the brightness of more superficial regions relative to the pre-ablation echogram. This region, which extends about 4.0 mm from the tissue surface, corresponds closely to the depth of the lesion when the same is examined in a photomicrograph.

In addition to masking underlying, non-lesioned tissue, the ordinarily skilled artisan will also appreciate that lesioned tissue is harder than non-lesioned tissue. As such, lesioned tissue deforms less under mechanical loads, such as tissue loading occurring naturally during the heartbeat cycle. With reference to Figure 7, for example, core region 74 will be harder than surrounding region 76, and surrounding region 76 will be harder than those portions of tissue 70 beyond lesion front 78. There are known techniques, including two- and three-dimensional elastography, to utilize this characteristic to monitor lesion formation. In some embodiments disclosed herein, these techniques are applied in only one dimension, obtaining elastographic stiffness only along that available beam direction.

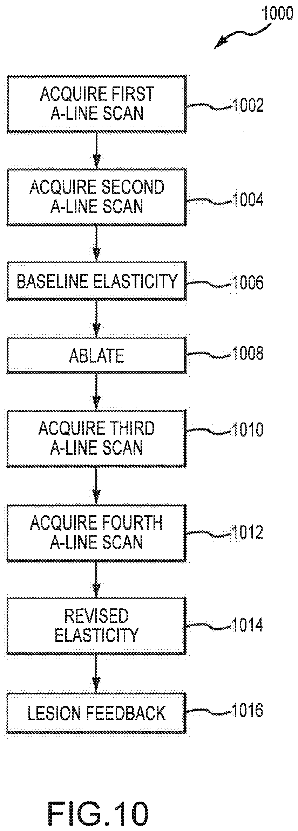

Figure 10 is a flowchart 1000 of representative steps that can be carried out to use this same elastographic characteristic to measure lesion formation using one-dimensional echograms (*e.g.,* A-line scans synchronized to the heartbeat to be along the same tissue path) by tracking the movement of acoustic reflectors 80, such as tissue features and/or stable microbubbles, within tissue 70 along beam path 34. In particular, the movement of acoustic reflectors 80 within tissue 70 along beam path 34 due to the heartbeat flexing of the heart wall can be used as a measure of lesion formation. Because the harder lesioned tissue will deform less than non-lesioned (or less-lesioned) tissue during the heartbeat cycle, the acoustic reflectors 80 within those regions will move less relative to each other along beam path 34. Although only a small number of uniformly distributed acoustic reflectors 80 are shown in Figure 7, it should be understood that this is only for the sake of illustration and that acoustic reflectors 80 will more typically be more widespread and randomly distributed through tissue 70, and in both ablated and unablated regions.

In block 1002, a first A-line scan echogram of tissue 70 is acquired. The point in the heartbeat cycle when this first echogram is acquired is referred to herein as the "first cardiac deformation state."

In block 1004, a second A-line scan echogram of tissue 70 is acquired. This second echogram is desirably acquired at a different point in the heartbeat cycle, referred to herein as the "second cardiac deformation state." It is advantageous for the first and second cardiac deformation states to be as different as possible (*e.g.*, maximum systole vs. maximum diastole) in order to maximize the relative deformation of tissue 70, and thus the movement of specific reflectors 80, between the two states.

A baseline tissue elasticity along the direction of beam path 34 is determined in block 1006 from the movement of acoustic reflectors 80 along beam path 34 between the first and second echograms acquired at the first and second cardiac deformation states before ablation stiffens any tissue.

Ablation, such as at least some incremental ablation, is carried out in block 1008. In blocks 1010 and 1012, after at least some ablation has occurred, a second, post-ablation pair of echograms is acquired, again taken at the first and second cardiac deformation states. These two new echograms, referred to herein as the third and fourth echograms, respectively, allow a revised elasticity along beam path 34 to be determined in block 1014, once again from the movement of acoustic reflectors 80 along beam path 34 between the first and second cardiac deformation states post-ablation. The ablation will typically reduce the movement of acoustic reflectors 80 relative to tip 22 due to reduced tissue elasticity.

In block 1016, feedback regarding the formation of lesion L is provided based upon the revised elasticity determined in block 1014. In certain aspects, the feedback is based upon a comparison of the revised elasticity to the baseline elasticity. In other aspects, the feedback is based upon a comparison of the revised elasticity to a desired elasticity that is known to correspond to the formation of a desired lesion.

It is contemplated that the foregoing steps can be repeated, either at the same location as lesion L continues to form, or at a different location. In the case where the foregoing steps are repeated, the revised elasticity can be compared to the baseline elasticity, the immediate prior revised elasticity (that is, each revised elasticity can be used as a new baseline elasticity for subsequent measurements), or to a composite or average thereof.

The ordinarily skilled artisan will also appreciate that lesions cause significant and long-lasting omnidirectional tissue shrinkage due, for example, to cross-linking and desiccation. This component of shrinkage can be detected by tracking acoustic reflectors 80 along just one direction. In addition to this actual physical shrinkage, however, there are at least two factors that will result in an echogram depicting apparent shrinkage in lesioned tissue. First, because the lesioned tissue is hotter than surrounding tissue, the speed of sound therethrough will be higher than the value assumed when taking echograms, usually about 1440 m/s. Second, because the lesioned tissue is harder than surrounding tissue, the speed of sound therethrough will be higher than the value assumed when taking echograms.

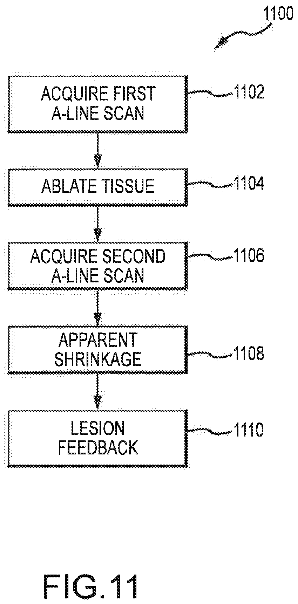

Figure 11 is a flowchart 1100 of representative steps that can be carried out to use this apparent shrinkage to measure lesion formation using one-dimensional echograms (*e.g.*, A-line scans). As with the embodiment discussed above in connection with Figure 10, this can be carried out by tracking the movement of acoustic reflectors 80 within tissue 70 along beam path 34. In the case of the embodiment of Figure 11, however, it is permanent shrinkage relative to a starting tissue thickness, which should be accompanied by reduced tissue elasticity, that is of interest. Thus, in connection with the method depicted in Figure 11, it is desirable for all echograms to be acquired at the same cardiac deformation state and for the tissue temperature to be approximately the same between A-line pinging scans.

In block 1102, a first A-line scan echogram of tissue 70 is acquired. Ablation, such as at least some incremental ablation, is carried out in block 1104. Following ablation, a second A-line scan echogram of tissue 70 is acquired in block 1106; as discussed above, the second A-line is desirable acquired at the same cardiac state, for example by synchronizing the transducer pinging to the heartbeat. In block 1108, the apparent shrinkage is determined from the apparent movement of acoustic reflectors 80 between the first and second echograms. Lesion feedback is provided in block 1110. It is contemplated that this shrinkage-based analysis can be done in parallel with the elasticity-based analysis discussed above to provide dual indications of the formation of lesion L.

Of course, the steps illustrated in Figure 11 can be repeated, either at the same location as lesion L continues to form, or at a different location. In the case where the foregoing steps are repeated, and unlike the embodiments previously described, apparent shrinkage will typically be determined relative to the initial (pre-lesioning) state of no apparent shrinkage, rather than relative to an immediately previous apparent shrinkage measurement, a composite or average, or the like.

In yet another aspect, lesion formation can be measured by monitoring resonant microbubbles. It is known that a forming lesion, as well as adjacent tissue, is populated by nucleating and nucleated microbubbles. These microbubbles include gas that was previously in solution; at higher temperatures, they can include water vapor and steam. The radius $r$ of a microbubble determines its resonant frequency $f_r$ under ultrasonic excita-

$$f_r = \frac{1}{2}\pi r \sqrt{\frac{Sa*b*\beta}{m}}$$

tion according to the equation ,
where $Sa$ is the microbubble adiabatic stiffness, $b$ is the reciprocal of the polytropic coefficient, $\beta$ is the surface tension coefficient, and $m$ is the mass of the system. Acoustic practitioners will appreciate that microbubbles typically also have harmonic resonance modes; it is contemplated that excitation can occur at a first frequency, with echoes received at a lower or higher frequency harmonic of the excitation frequency.

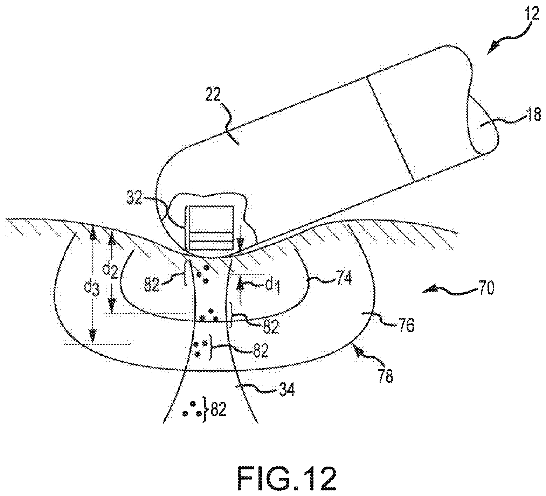

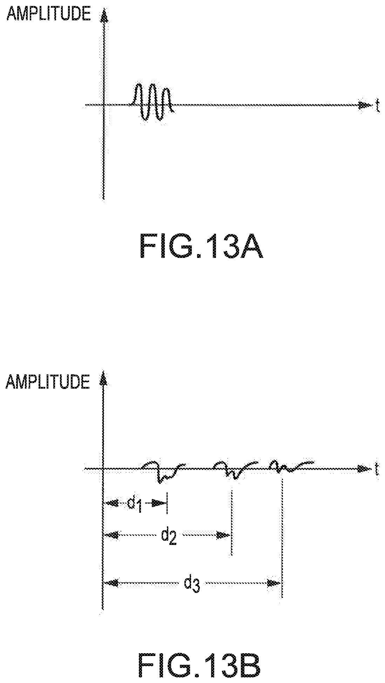

Figure 12 depicts microbubbles 82 forming in tissue 70 as lesion L forms. To detect these microbubbles, transducer 32 is operated to emit narrowband pulses, for example as shown in Figure 13a. The frequency of these pulses can be preset to correspond to the resonant frequency $f_r$ of certain radius microbubbles that are known (or, in embodiments, experimentally determined) to be characteristic of lesion formation and that excite the above-described harmonics. For example, the frequency of the narrowband pulses can be preset to correspond to the resonant frequency of microbubbles that are known to occur in a typical lesion resulting from a given ablation power, irrigation depth, and time. As discussed above, the microbubbles may respond at the excitation resonant frequency or at a lower or higher harmonic thereof.

It is also contemplated that, rather than using a fixed narrowband excitation frequency, a frequency ramped narrowband signal wherein several measurements, each at a different narrowband ramped frequency, can be employed.

Figure 13b is a representative returned echo plot of the narrowband pulses shown in Figure 13a for the arrangement depicted in Figure 12. Three echoes are shown, corresponding to the microbubbles 82 at depths $d_1$, $d_2$, and $d_3$ in Figure 12. These echoes can be interpreted as information about the distribution of the microbubbles 82 within tissue 70; by comparing the distribution of microbubbles 82 over time (comparisons can be made to the distribution of microbubbles 82 prior to any ablation, at an earlier time during ablation, or to a composite or average thereof), the progress of the formation of lesion L can be monitored. Further, by changing or sweeping the narrowband frequency (*e.g.,* by using a frequency ramped narrowband signal, as discussed above), one can scan for a variety of sizes of microbubbles 82.

In general, higher temperatures cause more microbubbles, and longer ablation times also cause some combining and growth of preexisting microbubbles. Thus, a bubble state, with known ablation power conditions, provides information relating to thermal exposure versus depth. Via bench calibration, this information relating to thermal exposure versus depth can be interpreted as a degree of lesioning versus depth.

The foregoing approaches to measuring lesion formation can be carried out, either singly or in any combination, by a processor incorporated into control unit 130. As used herein, the term processor refers to not only a single central processing unit ("CPU"), but also to a plurality of processing units, commonly referred to as a parallel processing environment. It should also be understood that the methods disclosed herein can be hardware and/or software implemented. Although the description above relates to providing a practitioner with feedback regarding lesion formation, the lesion formation information can also be used to automatically control the ablation process (*e.g.,* control unit 130 can be programmed to discontinue the application of ablative energy when lesion L reaches a desired depth d).

As another example, in addition or as an alternative to tracking the actual and/or apparent movement of acoustic reflectors along beam path 34, their lateral (*i.e.,* across beam path 34) cross-over frequency as they move through beam path 34 can be tracked as a measure of lesion formation by analogy to the tissue elasticity and tissue shrinkage methodologies disclosed herein.

As still another example, the several lesion monitoring methodologies discussed herein can not only be employed singly, but also in various combinations and/or weighted combinations.

All directional references (e.g., upper, lower, upward, downward, left, right, leftward, rightward, top, bottom, above, below, vertical, horizontal, clockwise, and counterclockwise) are only used for identification purposes to aid the reader's understanding of the present invention, and do not create limitations, particularly as to the position, orientation, or use of the invention. Joinder references (e.g., attached, coupled, connected, and the like) are to be construed broadly and may include intermediate members between a connection of elements and relative movement between elements. As such, joinder references do not necessarily infer that two elements are directly connected and in fixed relation to each other.

It is intended that all matter contained in the above description or shown in the accompanying drawings shall be interpreted as illustrative only and not limiting. Changes in detail or structure may be made without departing from the spirit of the invention as defined in the appended claims.

## Claims

1. A system for measuring lesion formation in a tissue, comprising:

a lesion analysis processor programmed to receive as input at least two A-line scan echograms of the tissue, to determine progress of a lesion forming in the tissue by comparing the at least two A-line scan echograms to each other, and to output feedback about the lesion, **characterized in that**
the at least two A-line scan echograms of the tissue are taken at common cardiac deformation states, and along common scan lines at different timings,
the lesion analysis processor is programmed to determine progress of the lesion forming in the tissue from the at least two A-line scan echograms by comparing a brightness of a first echogram of the at least two A-line scan echograms to a brightness of a second echogram of the at least two A-line scan echograms,
wherein the lesion analysis processor is programmed for
acquiring a first A-line scan electrogram of the tissue from an ultrasound imaging device operating at a first transmit power and a first gain, wherein the first A-line scan electrogram comprises a first line scan of the tissue from a surface of the tissue to a depth within the tissue;
acquiring a second A-line scan electrogram of the tissue from the ultrasound imaging device after acquiring the first A-line scan electrogram and after an ablation of the tissue, wherein the second A-line scan electrogram comprises a second scan line of the tissue from the surface of the tissue to the depth within the tissue;
identifying a region that appears darker in the second A-line scan electrogram than in the first A-line scan electrogram when the second A-line scan electrogram is acquired with the ultrasound imaging device operating at the first transmit power and the first gain;
increasing a brightness of the second A-line scan electrogram until the region that appears darker in the second A-line scan electrogram than in the first A-line scan electrogram appears as bright in the second A-line scan electrogram as it appears in the first A-line scan electrogram; and
after increasing the brightness of the second A-line scan electrogram:

identifying a region that appears brighter in the second A-line scan electrogram than it appears in the first A-line scan electrogram; and
providing feedback about a lesion forming in the tissue based upon the region that appears brighter in the second A-line scan electrogram than it appears in the first A-line scan electrogram.

2. The system according to claim 1, wherein increasing the brightness of the second A-line scan electrogram comprises increasing a receive gain of the ultrasound imaging device relative to the first gain prior to acquiring the second A-line scan electrogram, or wherein increasing a brightness of the second A-line scan electrogram comprises increasing transmit power of the ultrasound imaging device relative to the first transmit power prior to acquiring the second A-line scan electrogram.

3. The system according to claim 1, wherein providing feedback about a lesion forming in the tissue based upon the region that appears brighter in the second A-line scan electrogram than it appears in the first A-line scan electrogram comprises providing lesion depth information according to a depth of the region that appears brighter in the second A-line scan electrogram than it appears in the first A-line scan electrogram.

4. A system for measuring lesion formation in a tissue, comprising:

a lesion analysis processor programmed to receive as input at least two A-line scan echograms of the tissue, to determine progress of a lesion forming in the tissue by comparing the at least two A-line scan echograms to each other, and to output feedback about the lesion, **characterized in that**
the lesion analysis processor is programmed to determine progress of a lesion forming in the tissue from the at least two A-line scan echograms by analyzing changes in actual movement of acoustic reflectors within the tissue due to changes in tissue elasticity due to lesion formation using the at least two A-line scan echograms, or
the lesion analysis processor is programmed to determine progress of a lesion forming in the tissue from the at least two A-line scan echograms by analyzing apparent movement of acoustic reflectors within the tissue due to increases in acoustic velocity resulting from lesion formation using the at least two A-line scan echograms, or
the lesion analysis processor is programmed to determine progress of a lesion forming in the tissue from the at least two A-line scan echograms by analyzing changes in resonant microbubble distribution due to lesion formation using the at least two A-line scan echograms.

5. The system according to claim 4, wherein the lesion analysis processor is programmed for

acquiring a first A-line scan echogram of the cardiac tissue at a first cardiac deformation state;
acquiring a second A-line scan echogram of the cardiac tissue at a second cardiac deformation state;
computing a baseline elasticity of the cardiac tissue from the first A-line scan echogram and the second A-line scan echogram;
after an ablation of the cardiac tissue:

acquiring a third A-line scan echogram of the cardiac tissue at the first cardiac deformation state;
acquiring a fourth A-line scan echogram of the cardiac tissue at the second cardiac deformation state;
computing a revised elasticity of the cardiac tissue from the third A-line scan echogram and the fourth A-line scan echogram; and
providing feedback about a lesion forming in the cardiac tissue based upon the revised elasticity of the cardiac tissue, or

wherein providing feedback about a lesion forming in the cardiac tissue based upon the revised elasticity of the cardiac tissue comprises providing feedback about a lesion forming in the cardiac tissue based upon a comparison of the revised elasticity to the baseline elasticity, or
wherein providing feedback about a lesion forming in the cardiac tissue based upon the revised elasticity of the cardiac tissue comprises providing feedback about a lesion forming in the cardiac tissue based upon a comparison of the revised elasticity to a desired elasticity.

6. The system according to claim 5, wherein:

computing the baseline elasticity of the cardiac tissue comprises computing the baseline elasticity of the cardiac tissue based upon movement of acoustic reflectors within the cardiac tissue between the first A-line scan echogram and the second A-line scan echogram; and
computing the revised elasticity of the cardiac tissue comprises computing the revised elasticity of the cardiac tissue based upon movement of acoustic reflectors within the cardiac tissue between the third A-line scan echogram and the fourth A-line scan echogram.

7. The system according to claim 4, wherein the lesion analysis processor is programmed for

acquiring a first A-line scan echogram of the cardiac tissue;
and, after ablating the cardiac tissue:

acquiring a second A-line scan echogram of the cardiac tissue along a common tissue path relative to the first A-line scan echogram of the cardiac tissue;
determining an apparent shrinkage of the cardiac tissue along the A-line from the first A-line scan echogram and the second A-line scan echogram; and
providing feedback about a lesion forming in the cardiac tissue based upon the apparent shrinkage of the cardiac tissue.

8. The system according to claim 7, wherein determining the apparent shrinkage of the cardiac tissue from the first A-line scan echogram and the second A-line scan echogram comprises determining the apparent shrinkage based upon apparent movement of acoustic reflectors within the cardiac tissue between the first A-line scan echogram and the second A-line scan echogram.

9. The system according to claim 4, wherein the lesion analysis processor is programmed for

acquiring a first A-line scan echogram of the cardiac tissue at a first cardiac deformation state; and, after ablating the cardiac tissue:

acquiring a second A-line scan echogram of the cardiac tissue at the first cardiac deformation state; and
providing feedback about a lesion forming in the cardiac tissue by analyzing at least one of actual movement of acoustic reflectors within the cardiac tissue and apparent movement of acoustic reflectors within the cardiac tissue using at least the first A-line scan and the second A-line scan, or

providing feedback about a lesion forming in the cardiac tissue comprises providing feedback about the lesion forming in the cardiac tissue using apparent movement of acoustic reflectors due to increases in acoustic velocity within the cardiac tissue resulting from a lesion, or
providing feedback about a lesion forming in the cardiac tissue comprises providing feedback about the lesion forming in the cardiac tissue using actual movement of acoustic reflectors due to decreases in tissue elasticity within the cardiac tissue resulting from a lesion.

10. The system according to claim 4, wherein the lesion analysis processor is programmed for

emitting narrowband pulsed acoustic energy towards the tissue at a preset frequency, wherein the preset frequency comprises a resonant frequency of a microbubble characteristic of lesion formation;

detecting echoes of the emitted acoustic energy at one or more of the resonant frequency and harmonics of the resonant frequency; and providing feedback about a lesion forming in the tissue by analyzing a distribution of the microbubble characteristic of lesion formation within the tissue using the detected echoes of the emitted acoustic energy.

11. The system according to claim 10, further comprising:

after ablating the tissue, repeating the steps of:

emitting narrowband pulsed acoustic energy towards the tissue at a preset frequency; detecting echoes of the emitted acoustic energy; and analyzing a distribution of the microbubble characteristic of lesion formation within the tissue using the detected echoes of the emitted acoustic energy,

wherein providing feedback about a lesion forming in the tissue comprises analyzing a change in the distribution of the microbubble characteristic of lesion formation within the tissue from prior to ablating the tissue to after ablating the tissue.


**Patentansprüche**

1. System zum Messen einer Läsionbildung in einem Gewebe, mit:

einem Läsionanalyseprozessor, der programmiert ist zum Empfangen einer Eingabe von mindestens zwei A-Zeilenscanechogrammen des Gewebes zum Bestimmen eines Fortschritts einer Läsionbildung in dem Gewebe, indem die mindestens zwei A-Zeilenscanechogramme miteinander verglichen werden, und zum Ausgeben einer Rückmeldung über die Läsion, **dadurch gekennzeichnet, dass** die mindestens zwei A-Zeilenscanechogramme des Gewebes bei gemeinsamen Herzdeformierungszuständen genommen werden, und entlang gemeinsamer Abtastzeilen zu unterschiedlichen Zeiten, der Läsionanalyseprozessor programmiert ist zum Bestimmen eines Fortschritts der Läsionbildung in dem Gewebe aus den mindestens zwei A-Zeilenscanechogrammen, indem eine Helligkeit eines ersten Echogramms von den mindestens zwei A-Zeilenscanechogrammen mit einer Helligkeit eines zweiten Echogramms von den mindestens zwei A-Zeilenscanechogrammen verglichen wird, wobei der Läsionanalyseprozessor programmiert ist zum Erfassen eines ersten A-Zeilenscanelektrogramms des Gewebes von einer Ultraschallbildgebungsvorrichtung, die mit einer ersten Sendeleistung und einem ersten Gewinn arbeitet, wobei das erste A-Zeilenscanelektrogramm einen ersten Zeilenscan des Gewebes von einer Oberfläche des Gewebes bis zu einer Tiefe innerhalb des Gewebes enthält; Erfassen eines zweiten A-Zeilenscanelektrogramms des Gewebes von der Ultraschallbildgebungsvorrichtung nach dem Erfassen des ersten A-Zeilenscanelektrogramms und nach einer Ablation des Gewebes, wobei das zweite A-Zeilenscanelektrogramm eine zweite Scanzeile des Gewebes von der Oberfläche des Gewebes bis zu der Tiefe innerhalb des Gewebes enthält; Identifizieren einer Region, die dunkler erscheint in dem zweiten A-Zeilenscanelektrogramm als in dem ersten A-Zeilenscanelektrogramm, wenn das zweite A-Zeilenscanelektrogramm mit der Ultraschallbildgebungsvorrichtung erfasst wird, die mit der ersten Sendeleistung und dem ersten Gewinn arbeitet; Erhöhen einer Helligkeit des zweiten A-Zeilenscanelektrogramms bis die Region, die in dem zweiten A-Zeilenscanelektrogramm dunkler erscheint als in dem ersten A-Zeilenscanelektrogramm gleich hell in dem zweiten A-Zeilenscanelektrogramm erscheint, wie sie in dem ersten A-Zeilenscanelektrogramm erscheint; und nach dem Erhöhen der Helligkeit des zweiten A-Zeilenscanelektrogramms:

Identifizieren einer Region, die heller in dem zweiten A-Zeilenscanelektrogramm erscheint als in dem ersten A-Zeilenscanelektrogramm; und Bereitstellen einer Rückmeldung über eine Läsionbildung in dem Gewebe basierend auf der Region, die in dem zweiten A-Zeilenscanelektrogramm heller erscheint als in dem ersten A-Zeilenscanelektrogramm.

2. System nach Anspruch 1, bei dem das Erhöhen der Helligkeit des zweiten A-Zeilenscanelektrogramms ein Erhöhen eines Empfangsgewinns der Ultraschallbildgebungsvorrichtung relativ zu dem ersten Gewinn vor dem Erfassen des zweiten A-Zeilenscanelektrogramms aufweist, oder wobei das Erhöhen einer Helligkeit des zweiten A-Zeilenscanelektrogramms ein Erhöhen der Sendeleistung der Ultraschallbildgebungsvorrichtung relativ zu der ersten Sendeleistung vor dem Erfassen des zweiten A-Zeilenscanelektrogramms aufweist.

**3.** System nach Anspruch 1, bei dem das Bereitstellen der Rückmeldung über eine Läsionbildung in dem Gewebe basierend auf der Region, die in dem zweiten A-Zeilenscanelektrogramm heller erscheint als in dem ersten A-Zeilenscanelektrogramm, ein Bereitstellen einer Läsiontiefeninformation aufweist gemäß einer Tiefe der Region, die in dem zweiten A-Zeilenscanelektrogramm heller erscheint als in dem ersten A-Zeilenscanelektrogramm.

**4.** System zum Messen einer Läsionbildung in einem Gewebe, mit:

einem Läsionanalyseprozessor, der programmiert ist zum Empfangen von mindestens zwei A-Zeilenscanechogrammen des Gewebes als Eingabe, zum Bestimmen eines Fortschritts einer Läsionbildung in dem Gewebe, indem die mindestens zwei A-Zeilenscanechogramme miteinander verglichen werden, und zum Ausgeben einer Rückmeldung über die Läsion, **dadurch gekennzeichnet, dass**

der Läsionanalyseprozessor programmiert ist zum Bestimmen eines Fortschritts einer Läsionbildung in dem Gewebe aus den mindestens zwei A-Zeilenscanechogrammen durch Analysieren von Änderungen der tatsächlichen Bewegung von akustischen Reflektoren innerhalb des Gewebes aufgrund von Änderungen in der Gewebeelastizität aufgrund der Läsionbildung, indem die mindestens zwei A-Zeilenscanelektrogramme verwendet werden, oder

der Läsionanalyseprozessor programmiert ist zum Bestimmen eines Fortschritts einer Läsionbildung in dem Gewebe aus den mindestens zwei A-Zeilenscaneechogrammen, indem eine offensichtliche Bewegung der akustischen Reflektoren innerhalb des Gewebes aufgrund einer Erhöhung einer akustischen Geschwindigkeit analysiert wird, die von der Läsionbildung her resultiert, indem die mindestens zwei A-Zeilenscanechogramme verwendet werden, oder

der Läsionanalyseprozessor programmiert ist zum Bestimmen eines Fortschritts einer Läsionbildung in dem Gewebe aus den mindestens zwei A-Zeilenscanechogrammen durch Analysieren von Änderungen in einer Verteilung von Mikrobläschen in Resonanz aufgrund der Läsionbildung, indem die mindestens zwei A-Zeilenscanechogramme verwendet werden.

**5.** System nach Anspruch 4, bei dem der Läsionanalyseprozessor programmiert ist zum

Erfassen eines ersten A- Zeilenscanechogramms des Herzgewebes bei einem ersten Herzverformungszustand;
Erfassen eines zweiten A- Zeilenscanechogramms des Herzgewebes bei einem zweiten Herzverformungszustand;
Berechnen einer Grundelastizität des Herzgewebes aus dem ersten A-Zeilenscanechogramm und dem zweiten A-Zeilenscanechogramm;
nach einer Ablation des Herzgewebes:

Erfassen eines dritten A-Zeilenscanechogramms des Herzgewebes bei dem ersten Herzverformungszustand;
Erfassen eines vierten A-Zeilenscanechogramms des Herzgewebes bei dem zweiten Herzverformungszustand;
Berechnen einer revidierten Elastizität des Herzgewebes aus dem dritten A-Zeilenscanechogramm und dem vierten A-Zeilenscanechogramm; und
Bereitstellen einer Rückmeldung über eine Läsionbildung in dem Herzgewebe basierend auf der revidierten Elastizität des Herzgewebes, oder

wobei das Bereitstellen der Rückmeldung über eine Läsionbildung in dem Herzgewebe basierend auf der revidierten Elastizität des Herzgewebes ein Bereitstellen einer Rückmeldung über eine Läsionbildung in dem Herzgewebe basierend auf einem Vergleich der revidierten Elastizität mit der Grundelastizität aufweist, oder

wobei das Bereitstellen der Rückmeldung über eine Läsionbildung in dem Herzgewebe basierend auf der revidierten Elastizität des Herzgewebes ein Bereitstellen einer Rückmeldung über eine Läsionbildung in dem Herzgewebe basierend auf einem Vergleich der revidierten Elastizität mit einer gewünschten Elastizität aufweist.

**6.** System nach Anspruch 5, bei dem:

das Berechnen der Grundelastizität des Herzgewebes ein Berechnen der Grundelastizität des Herzgewebes basierend auf einer Bewegung akustischer Reflektoren innerhalb des Herzgewebes zwischen dem ersten A-Zeilenscanechogramm und dem zweiten A-Zeilenscanechogramm aufweist; und
das Berechnen der revidierten Elastizität des Herzgewebes ein Berechnen der revidierten Elastizität des Herzgewebes basierend auf einer Bewegung akustischer Reflektoren innerhalb des Herzgewebes zwischen dem dritten A-Zeilenscanechogramm und dem vierten A-Zeilenscanechogramm aufweist.

**7.** System nach Anspruch 4, bei dem der Läsionanalyseprozessor programmiert ist zum

Erfassen eines ersten A-Zeilenscanechogramms des Herzgewebes; und, nach einer Ablation des Herzgewebes:

Erfassen eines zweiten A-Zeilenscanechogramms des Herzgewebes entlang eines gemeinsamen Gewebepfads relativ zu dem ersten A-Zeilenscanechogramm des Herzgewebes;
Bestimmen einer sichtbaren Schrumpfung des Herzgewebes entlang der A-Zeile von dem ersten A-Zeilenscanechogramm und dem zweiten A-Zeilenscanechogramm; und
Bereitstellen einer Rückmeldung über eine Läsionbildung in dem Herzgewebe basierend auf der sichtbaren Schrumpfung des Herzgewebes.

8. System nach Anspruch 7, bei dem das Bestimmen der sichtbaren Schrumpfung des Herzgewebes von dem ersten A-Zeilenscanechogramm und dem zweiten A-Zeilenscanechogramm ein Bestimmen der sichtbaren Schrumpfung basierend auf der sichtbaren Bewegung der akustischen Reflektoren innerhalb des Herzgewebes zwischen dem ersten A-Zeilenscanechogramm und dem zweiten A-Zeilenscanechogramm aufweist.

9. System nach Anspruch 4, bei dem der Läsionanalyseprozessor programmiert ist zum

Erfassen eines ersten A-Zeilenscanechogramms des Herzgewebes bei einem ersten Herzverformungszustand; und
nach einer Ablation des Herzgewebes:

Erfassen eines zweiten A-Zeilenscanechogramms des Herzgewebes bei dem ersten Herzverformungszustand; und
Bereitstellen einer Rückmeldung über eine Läsionbildung in dem Herzgewebe durch Analysieren von mindestens einer tatsächlichen Bewegung von akustischen Reflektoren innerhalb des Herzgewebes und einer sichtbaren Bewegung der akustischen Reflektoren innerhalb des Herzgewebes unter Verwendung von mindestens des ersten A-Zeilenscans und des zweiten A-Zeilenscans, oder
das Bereitstellen der Rückmeldung über eine Läsionbildung in dem Herzgewebe ein Bereitstellen einer Rückmeldung über die Läsionbildung in dem Herzgewebe aufweist unter Verwendung der sichtbaren Bewegung von akustischen Reflektoren aufgrund der Erhöhung der akustischen Geschwindigkeit innerhalb des Herzgewebes, die von einer Läsion her resultiert, oder
das Bereitstellen einer Rückmeldung über eine Läsionbildung in dem Herzgewebe ein Bereitstellen einer Rückmeldung über die Läsionbildung in dem Herzgewebe aufweist unter Verwendung der tatsächlichen Bewegung der akustischen Reflektoren aufgrund einer Reduzierung der Gewebeelastizität innerhalb des Herzgewebes, die von einer Läsion her resultiert.

10. System nach Anspruch 4, bei dem der Läsionanalyseprozessor programmiert ist zum

Aussenden einer schmalbandigen gepulsten akustischen Energie in Richtung des Gewebes mit einer vorbestimmten Frequenz, wobei die vorbestimmte Frequenz eine Resonanzfrequenz einer Mikroblasencharakteristik einer Läsionbildung aufweist;
Detektieren von Echos der ausgesendeten akustischen Energie bei einer oder mehreren von der Resonanzfrequenz und Harmonischen der Resonanzfrequenz; und
Bereitstellen einer Rückmeldung über eine Läsionbildung in dem Gewebe durch Analysieren einer Verteilung der Mikroblasencharakteristik der Läsionbildung innerhalb des Gewebes unter Verwendung der detektierten Echos der ausgesendeten akustischen Energie.

11. System nach Anspruch 10, ferner mit: nach einer Ablation des Gewebes, Wiederholen der Schritte:

Aussenden einer schmalbandigen gepulsten akustischen Energie in Richtung des Gewebes bei einer vorbestimmten Frequenz;
Detektieren von Echos der ausgesendeten akustischen Energie; und
Analysieren einer Verteilung der Mikroblasencharakteristik der Läsionbildung innerhalb des Gewebes unter Verwendung der detektierten Echos der ausgesendeten akustischen Energie, wobei das Bereitstellen der Rückmeldung über eine Läsionbildung in dem Gewebe ein Analysieren einer Änderung der Verteilung der Mikroblasencharakteristik der Läsionbildung innerhalb des Gewebes von vor Ablation des Gewebes bis nach Ablation des Gewebes aufweist.

**Revendications**

1. Système de mesure de la formation de lésion dans un tissu, comprenant :

un processeur d'analyse de lésion programmé pour recevoir en entrée au moins deux échogrammes à balayage linéaire du tissu, pour déterminer une progression d'une lésion se formant dans le tissu en comparant lesdits au moins deux échogrammes à balayage linéaire l'un à l'autre, et pour émettre une rétroaction sur la lésion,

**caractérisé en ce que**

lesdits au moins deux échogrammes à balayage linéaire du tissu sont pris à des états de déformation cardiaque communs et le long de lignes de balayage communes à des moments différents,

le processeur d'analyse de lésion est programmé pour déterminer une progression de la lésion formant dans le tissu à partir desdits au moins deux échogrammes à balayage linéaire en comparant une luminosité d'un premier échogramme desdits au moins deux échogrammes à balayage linéaire à une luminosité d'un second échogramme desdits au moins deux échogrammes à balayage linéaire,

dans lequel le processeur d'analyse de lésion est programmé pour

acquérir un premier électrogramme à balayage linéaire du tissu à partir d'un dispositif d'imagerie à ultrasons fonctionnant à une première puissance d'émission et à un premier gain, dans lequel le premier électrogramme à balayage linéaire comprend un premier balayage linéaire du tissu depuis une surface du tissu jusqu'à une profondeur à l'intérieur du tissu ;

acquérir un second électrogramme à balayage linéaire du tissu à partir du dispositif d'imagerie à ultrasons après l'acquisition du premier électrogramme à balayage linéaire et après une ablation du tissu, dans lequel le second électrogramme à balayage linéaire comprend une seconde ligne de balayage du tissu depuis la surface du tissu jusqu'à la profondeur à l'intérieur du tissu ;

identifier une région qui semble plus sombre dans le second électrogramme à balayage linéaire que dans le premier électrogramme à balayage linéaire lorsque le second électrogramme à balayage linéaire est acquis avec le dispositif d'imagerie à ultrasons fonctionnant à la première puissance d'émission et au premier gain ;

augmenter une luminosité du second électrogramme à balayage linéaire jusqu'à ce que la région qui semble plus sombre dans le second électrogramme à balayage linéaire que dans le premier électrogramme à balayage linéaire apparaisse aussi claire dans le second électrogramme à balayage linéaire que dans le premier électrogramme à balayage linéaire ; et

après l'augmentation de la luminosité du second électrogramme à balayage linéaire :

identifier une région qui apparaît plus claire dans le second électrogramme à balayage linéaire que dans le premier électrogramme à balayage linéaire ; et

fournir une rétroaction sur la formation d'une lésion dans le tissu sur la base de la région qui apparaît plus claire dans le second électrogramme à balayage linéaire que dans le premier électrogramme à balayage linéaire.

2. Système selon la revendication 1, dans lequel l'augmentation de la luminosité du second électrogramme à balayage linéaire comprend l'augmentation d'un gain de réception du dispositif d'imagerie à ultrasons par rapport au premier gain avant l'acquisition du second électrogramme à balayage linéaire, ou

dans lequel l'augmentation d'une luminosité du second électrogramme à balayage linéaire comprend une augmentation d'une puissance d'émission du dispositif d'imagerie à ultrasons par rapport à la première puissance d'émission avant l'acquisition du second électrogramme à balayage linéaire.

3. Système selon la revendication 1, dans lequel la fourniture d'une rétroaction sur une lésion formant dans le tissu en fonction de la région qui apparaît plus claire dans le seconde électrogramme à balayage linéaire que dans le premier électrogramme à balayage linéaire comprend la fourniture d'une information de profondeur de lésion en fonction d'une profondeur de la région qui apparaît plus claire dans le second électrogramme à balayage linéaire que dans le premier électrogramme à balayage linéaire.

4. Système de mesure de formation de lésions dans un tissu, comprenant :

un processeur d'analyse de lésion programmé pour recevoir en entrée au moins deux échogrammes à balayage linéaire du tissu, pour déterminer une progression d'une lésion se formant dans le tissu en comparant lesdits au moins deux échogrammes à balayage linéaire l'un à l'autre, et pour émettre une rétroaction sur la lésion,

**caractérisé en ce que**

le processeur d'analyse de lésion est programmé pour déterminer une progression d'une formation de lésion dans le tissu à partir dudit au moins deux échogrammes à balayage linéaire en analysant les changements dans le mouvement réel des réflecteurs acoustiques à l'intérieur du tissu en raison des changements dans l'élasticité du tissu dus à la formation de lésion à l'aide d'au moins deux échogrammes à balayage linéaire, ou

le processeur d'analyse de lésion est programmé pour déterminer la progression d'une formation de lésion dans le tissu à partir dudit au moins deux échogrammes à balayage linéaire en analysant un mouvement apparent de réflecteurs acoustiques dans le tissu en raison de l'augmentation de la vitesse acoustique résultant de la formation de lésion à l'aide desdits au moins deux échogrammes à balayage linéaire, ou le processeur d'analyse de lésion est programmé pour déterminer une progression d'une formation de lésion dans le tissu à partir desdits au moins deux échogrammes à balayage linéaire en analysant les changements dans une distribution de microbulle résonnante due à la formation de lésion à l'aide d'au moins deux échogrammes à balayage linéaire.

5. Système selon la revendication 4, dans lequel le processeur d'analyse de lésion est programmé pour

   acquérir un premier échogramme à balayage linéaire du tissu cardiaque à un premier état de déformation cardiaque ;
   acquérir un second échogramme à balayage linéaire du tissu cardiaque à un second état de déformation cardiaque ;
   calculer l'élasticité de base du tissu cardiaque à partir du premier échogramme à balayage linéaire et du second échogramme à balayage linéaire ;
   après une ablation du tissu cardiaque :

      acquérir un troisième échogramme à balayage linéaire du tissu cardiaque au premier état de déformation cardiaque ;
      acquérir un quatrième échogramme à balayage linéaire du tissu cardiaque au second état de déformation cardiaque ;
      calculer une élasticité révisée du tissu cardiaque à partir du troisième échogramme à balayage linéaire et du quatrième échogramme à balayage linéaire ; et
      fournir une rétroaction sur une lésion formant dans le tissu cardiaque sur la base de l'élasticité révisée du tissu cardiaque, ou
      dans lequel la fourniture d'une rétroaction sur une lésion formant dans le tissu cardiaque basée sur l'élasticité révisée du tissu cardiaque comprend la fourniture d'une rétroaction sur une formation de lésion dans le tissu cardiaque en fonction d'une comparaison de l'élasticité révisée avec l'élasticité de base, ou
      dans lequel la fourniture d'une rétroaction sur une formation de lésion dans le tissu cardiaque en fonction de l'élasticité révisée du tissu cardiaque comprend la fourniture d'une rétroaction sur une formation de lésion dans le tissu cardiaque en fonction d'une comparaison entre l'élasticité révisée et l'élasticité souhaitée.

6. Système selon la revendication 5, dans lequel :

   le calcul de l'élasticité de base du tissu cardiaque comprend le calcul de l'élasticité de base du tissu cardiaque sur la base du mouvement des réflecteurs acoustiques dans le tissu cardiaque entre le premier échogramme à balayage linéaire et le second échogramme à balayage linéaire ; et
   le calcul de l'élasticité révisée du tissu cardiaque comprend le calcul de l'élasticité révisée du tissu cardiaque sur la base du mouvement des réflecteurs acoustiques dans le tissu cardiaque entre le troisième échogramme à balayage linéaire et le quatrième échogramme à balayage linéaire.

7. Système selon la revendication 4, dans lequel le processeur d'analyse de lésion est programmé pour

   acquérir un premier échogramme à balayage linéaire du tissu cardiaque ;
   et, après ablater le tissu cardiaque :

      acquérir un second échogramme à balayage linéaire du tissu cardiaque le long d'un parcours de tissu commun par rapport au premier échogramme à balayage linéaire du tissu cardiaque ;
      déterminer un retrait apparent du tissu cardiaque le long de la lignée à partir du premier échogramme à balayage linéaire et du second échogramme à balayage linéaire ; et
      fournir une rétroaction sur une formation de lésion dans le tissu cardiaque sur la base du rétrécissement apparent du tissu cardiaque.

8. Système selon la revendication 7, dans lequel la détermination du rétrécissement apparent du tissu cardiaque à partir du premier échogramme à balayage linéaire et du second échogramme à balayage linéaire comprend la détermination du rétrécissement apparent sur la base du mouvement apparent des réflecteurs acoustiques dans le tissu cardiaque entre le premier échogramme à balayage linéaire et le second échogramme à balayage linéaire.

9. Système selon la revendication 4, dans lequel le processeur d'analyse de lésion est programmé pour

   acquérir un premier échogramme à balayage linéaire du tissu cardiaque à un premier état de déformation cardiaque ;

et, après ablater le tissu cardiaque :

acquérir un second échogramme à balayage linéaire du tissu cardiaque au premier état de déformation cardiaque ; et

fournir une rétroaction sur une lésion formant dans le tissu cardiaque en analysant au moins un mouvement parmi un mouvement réel de réflecteurs acoustiques dans le tissu cardiaque et un mouvement apparent de réflecteurs acoustiques dans le tissu cardiaque en utilisant au moins le premier balayage linéaire et le second balayage linéaire, ou

fournir une rétroaction sur la formation de lésion dans le tissu cardiaque consiste à fournir une rétroaction sur la formation de lésion dans le tissu cardiaque en utilisant un mouvement apparent des réflecteurs acoustiques dû à l'augmentation de la vitesse acoustique dans le tissu cardiaque résultant d'une lésion, ou

fournir une rétroaction sur la formation d'une lésion dans le tissu cardiaque consiste à fournir une rétroaction sur la formation de lésion dans le tissu cardiaque en utilisant un mouvement réel des réflecteurs acoustiques dû à la diminution de l'élasticité de tissu dans le tissu cardiaque résultant d'une lésion.

**10.** Système selon la revendication 4, dans lequel le processeur d'analyse de lésion est programmé pour

émettre une énergie acoustique pulsée à bande étroite vers le tissu à une fréquence prédéfinie, dans lequel la fréquence prédéfinie comprend une fréquence de résonance d'une caractéristique de microbulle de formation de lésion ;

détecter des échos de l'énergie acoustique émise à une ou plusieurs des fréquences de résonance et des harmoniques de la fréquence de résonance ; et

fournir une rétroaction sur la formation d'une lésion dans le tissu en analysant une distribution de la caractéristique de microbulle de formation de lésion dans le tissu à l'aide des échos détectés de l'énergie acoustique émise.

**11.** Système selon la revendication 10, comprenant en outre :

après l'ablation du tissu, répéter les étapes consistant à :

émettre une énergie acoustique pulsée à bande étroite vers le tissu à une fréquence prédéfinie ;

détecter les échos de l'énergie acoustique émise ; et

analyser une distribution de la caractéristique de microbulle de lésion formant dans le tissu à l'aide des échos détectés de l'énergie acoustique émise,

dans lequel la fourniture d'une rétroaction sur une lésion formant dans le tissu comprend l'analyse d'un changement dans la distribution de la caractéristique de microbulle de lésion formant dans le tissu, avant l'ablation du tissu et après l'ablation du tissu.

FIG.1

FIG.2

FIG.3

FIG.4

22""

30""

32""

30""

30""

32""

32""

FIG.5

22''''

32''''

32''''

32''''

32''''

FIG.6

FIG.7

EP 3 226 773 B1

800

```
┌─────────────────────┐
│   ACQUIRE FIRST     │───── 802
│     ECHOGRAM        │
└─────────────────────┘
          │
          ▼
┌─────────────────────┐
│    ABLATE TISSUE    │───── 803
└─────────────────────┘
          │
          ▼
┌─────────────────────┐
│  ACQUIRE SECOND     │───── 804
│     ECHOGRAM        │
└─────────────────────┘
          │
          ▼
┌─────────────────────┐
│  IDENTIFY DARKER    │───── 806
│      REGION         │
└─────────────────────┘
          │
          ▼
┌─────────────────────┐
│     INCREASE        │───── 808
│    BRIGHTNESS       │
└─────────────────────┘
          │
          ▼
┌─────────────────────┐
│  IDENTIFY BRIGHTER  │───── 810
│      REGION         │
└─────────────────────┘
          │
          ▼
┌─────────────────────┐
│   PROVIDE LESION    │───── 812
│     FEEDBACK        │
└─────────────────────┘
```

# FIG.8

23

FIG.9A

FIG.9B

1000

ACQUIRE FIRST
A-LINE SCAN —1002

↓

ACQUIRE SECOND
A-LINE SCAN —1004

↓

BASELINE ELASTICITY —1006

↓

ABLATE —1008

↓

ACQUIRE THIRD
A-LINE SCAN —1010

↓

ACQUIRE FOURTH
A-LINE SCAN —1012

↓

REVISED
ELASTICITY —1014

↓

LESION FEEDBACK —1016

# FIG.10

1100

ACQUIRE FIRST
A-LINE SCAN —1102

ABLATE TISSUE —1104

ACQUIRE SECOND
A-LINE SCAN —1106

APPARENT
SHRINKAGE —1108

LESION
FEEDBACK —1110

FIG.11

FIG.12

FIG.13A

FIG.13B

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20100113985 A1
- US 20100198065 A1
- US 20140081262 A1
- US 20120265069 A1
- US 20120265069